# EUROPEAN PATENT APPLICATION

(11) **EP 3 988 647 A1**
(43) Date of publication of application: **27.04.2022**
(21) Application number: 20827697.2
(22) Date of filing: 18.06.2020
(51) Int. Cl.: C12N 5/074

(54) **HETEROGENEOUS STEM CELL POPULATION, PREPARATION METHOD THEREFOR AND USE THEREOF**

(30) Priority: 18.06.2019 CN 201910527634
(71) Applicant: Institute of Basic Medical Sciences, Chinese Academy of Medical Sciences, Beijing 100005 (CN)
(72) Inventor: ZHAO, Chunhua, Beijing 100005 (CN)
(74) Representative: Danner, Stefan
(86) International application number: PCT/CN2020/096750
(87) International publication number: WO 2020/253763

(57) **Abstract**

Disclosed are a heterogeneous stem cell population, a preparation method therefor, and the use thereof. Specifically, disclosed is a heterogeneous stem cell population, characterized in that stem cells in the heterogeneous stem cell population express stemness genes MYC, KLF4, GMNN, SOX2 and NANOG, and in the heterogeneous stem cell population, the ratio of stem cells expressing CD146 is 1%-50%.

## Description

### FIELD OF THE INVENTION

The present invention relates to a new heterogeneous stem cell population, characterized in that it is a stem cell population found in adult tissues retained after the stage of embryonic development, expressing the totipotent genes c-Myc, Gmnn and Klf4, and having an expression rate of 1-100% for CD146. The present invention also relates to a preparation method and the use of said heterogeneous stem cell population.

### BACKGROUND OF THE INVENTION

Mesenchymal stem cells (MSCs), also known as pluripotent mesenchymal stromal cells, constitute a heterogeneous cell population (Uccelli et al., 2008). The discovery of MSC is usually attributed to the work of A.J. In the late 1960s. Friedenstein and colleagues observed that culturing human bone marrow (BM) cell suspensions in plastic petri dishes resulted in a gradual loss of hematopoietic cells, which facilitated the proliferation of adherent fibroblast-like cell colonies that were capable of differentiating into adipocytes *in vitro* (Friedenstein et al., 1968) or *in vivo* (Friedenstein et al., 1974), chondrocytes and osteocytes.

The acronym "MSC" became popular after the work of A.I. In an article submitted in 1991, Caplan et al. proposed that in adult BM, stem cell populations can differentiate into different types of tissues derived from the mesodermal lineage (Caplan, 1991). They called these cells "mesenchymal stem cells". Later, Pittenger clearly demonstrated the multidirectional differentiation ability of MSCs (Pittenger et al., 1999). From the results of these groundbreaking studies, it can be seen that cultured and expanded MSCs have become the subject of many studies, even though the precise characterization of these cells remains to be elucidated, and no standardized protocols have been established to apply in different laboratories.

Therefore, the reported data are sometimes controversial, and many aspects of MSC biology are still unclear due to the diversity of isolation procedures, culture methods and the choice of tissue sources. In order to better clarify the controversy surrounding MSCs, the concept of mesenchymal stem cell system is proposed, which consists of all MSCs from different stages of embryonic development, from post-embryonic stem cells to progenitor cells. At the top of the MSC system are embryonic-like stem cells, which remain in many tissues even after the fetus is formed, being defined as post-embryonic pluripotent stem cells (PSCs).

PSCs from a variety of human fetal and adult tissues were identified and it was demonstrated that these cells can produce endothelial, hepatic epithelial, neuronal, hematopoietic, adipogenic and osteoblast cell lines. PSCs have now been confirmed by other groups. In 2010, Kuroda Y et al. demonstrated at the single-cell level that adult mesenchymal stem cells (MSCs) contain a unique type of stem cells that are capable of producing cells with the characteristics of all three germ layers. These cells are called multilineage-differentiating stress enduring (Muse) cells. A highly purified Muse cell population was isolated from the adipose tissue and was reported to spontaneously differentiate into mesenchymal, endodermal and ectodermal cell lineages.

All these studies indicate the presence of PSCs in adult tissues. It is assumed that other cells in the MSC system such as pericytes and general MSCs are derivatives of PSCs. The definition of PSC is based on the perspective of stem cell differentiation. Through years of research on this cell type, it is strongly advocated the idea of defining stem cells by their functions. Here, PSC were defined as culture-activated post-embryonic subpluripotent stem cells (CAPPSCs), which have three important biological characteristics: stemness properties including pluripotency and self-renewal, low immunogenicity and immunomodulatory function, as well as the maintenance of microenvironment and tissues.

### SUMMARY OF THE INVENTION

The inventor's research has found that the new stem cell population provided herein is different from the previously reported MSCs. It in that it is a mixed heterogeneous stem cell population with stronger stemness; it is in an euchromatic state with epigenetic pluripotency and expresses pluripotency markers MYC, KLF4 and GMNN. Most of the genes related to germ layer specification are modified by H3K4me3 or co-modified by H3K4me3 and H3K27me3. Using single-cell RNA-seq to analyze the differentiation process of new stem cells into functional hepatocytes, it was also found that in the very early stages of differentiation, the expression of genes related to early differentiation of the three germ layers are all successively up-regulated, while the expression of genes associated with the differentiation of other germ layers are down-regulated after the cells are directed to differentiate into the liver lineage.

In this study, single-cell analysis has shown that the new stem cell population isolated and cultured *in vitro* expresses the totipotent genes c-Myc, Gmnn and Klf4; expresses genes related to the early development of the three germ layers, such as Nes and Hes1 for the ectoderm, Pdgfra and Gsc for the mesoderm and endoderm, and Hhex and Sox17 restrictively for the endoderm; has high expression of ATF5, Tle3, Hnf4a and Krt18, which are key genes for the development of endodermal organ liver, and very weak expression of Mesp2, Gata4, Hand1 and Tbx6 for the mesoderm, but has significant expression of osteogenic differentiation related genes Wnt5a, Runx2 and TAZ, significant expression of Cebpb, Cebpd, Gsk3a and Gsk3b, which are upstream regulatory genes related to adipogenic differentiation, moderate expression of Mapk7, and low or no expression of Pparg and Cebpa, the key transcription factors for adipogenic differentiation.

Interestingly, it has been found that Tjp1, Ctnnb1, Cdh2, Fn1, Vim, Zeb1 and Twist1, key genes related to epithelial-mesenchymal transition, are all significantly expressed in the new stem cells, indicating they are in the middle stage of epithelial-mesenchymal transition, and capable of rapid functional conversion in response to different microenvironmental signals.

A heterogeneous stem cell population is provided in the present invention, characterized in that the stem cells contained therein express stemness genes MYC, KLF4, GMNN, SOX2 and NANOG, the ratio of stem cells expressing CD146 is 1-100%, and preferably the ratio of stem cells expressing CD146 is 1-50%.

In one aspect, while sub-cultured in medium containing 50%-99% DMEM/F12, 0.1-30ng/ml epidermal growth factor, 0.1-2% B27 and 0.1-10% FBS for 1, 2, 3, 4, 5, 6, 7, 8, 9, 10 or more than 10 passages, the ratio of stem cells expressing CD146 is 1-99%, and preferably, the ratio of stem cells expressing CD146 is 1-50%. In another aspect, when sub-cultured in medium containing FBS for 1, 2, 3, 4, 5, 6, 7, 8, 9, 10 or more passages, the ratio of stem cells expressing CD146 is 50-100%, and preferably said ratio is 55-100%.

Further, the heterogeneous stem cell population provided by the present invention is characterized in that the expression levels of stemness genes MYC, KLF4, GMNN, SOX2 and NANOG in stem cells with weakly positive expression of CD146 (CD146+, wherein the ratio of stem cells expressing CD146 being 1-50%, excluding 50%) are significantly higher than those in stem cells with strongly positive expression of CD146 (CD146+++, wherein the ratio of stem cells expressing CD146 being 50-100%).

A method for inducing the heterogeneous stem cell population provided by the present invention to differentiate into reticular vascular endothelial cells *in vitro* is described including inducing the heterogeneous stem cell population provided by the present invention to differentiate into reticular vascular endothelial cells in a culture medium containing 50%-99% DMEM/F12, 0.1-30 ng/ml epidermal growth factor, 0.1-2% B27 and 0.1-10% FBS.

A method for promoting T cell activation *in vitro* is demonstrated, including a step of co-culturing the heterogeneous stem cell population provided by the present invention and isolated PBMCs in a culture medium containing LPS.

A method for inhibiting T cell activation *in vitro* is illustrated, including a step of co-culturing the heterogeneous stem cell population provided by the present invention and isolated PBMCs in a culture medium containing PolylC or IFN-γ+TNF-α (I+T).

The present invention proposes the use of the heterogeneous stem cell of the invention in the preparation of a medicament for repairing tissue damage, especially hepatocyte-related liver damage. The present invention promotes the use of the heterogeneous stem cell population in the preparation of a medicament for treating cGVHD.

A method for preparing the heterogeneous stem cell population is provided by the present invention, including the following steps:
(1) obtaining a stem cell population;
(2) culturing the stem cell population obtained in step (1) in medium containing 50%-99% DMEM/F12, 0.1-30 ng/ml epidermal growth factor, 0.1-2% B27 and 0.1-10% FBS.

Moreover, the present invention relates to the use of the heterogeneous stem cell population according to the present invention in the preparation of a reagent for constructing the blood-brain barrier.

Furthermore, the present invention relates to the use of the heterogeneous stem cell population according to the present invention in the preparation of a reagent for inducing the differentiation of beige adipocytes.

Additionally, the inventor has found that a small molecule CZ can induce the new stem cell population to differentiate into an anti-inflammatory stem cell population MSC2. Combining with the results of *in vitro* experiments, single-cell sequencing was used to further study this transformation of the new stem cell population. After CZ treatment, the cells enter an activation state with anti-inflammatory effects similar to MSC2, which can be used in the clinical treatment of autoimmune diseases.

Therefore, the present invention further provides a novel method for inducing the heterogeneous stem cell population identified into an anti-inflammatory stem cell population *in vitro,* including contacting the heterogeneous stem cell population of the present invention with the small molecule CZ. In another aspect, the present invention provides use of the anti-inflammatory stem cell population obtained by the above method in the preparation of a medicament for treating autoimmune diseases.

### BRIEF DESCRIPTION OF THE DRAWINGS

**Figure 1A** shows the gene expression levels of MYC, KLF4, GMNN, SOX2 and NANOG in the heterogeneous stem cell populations obtained by the mesenchymal stem cell culture method of the present invention (MSC-AB) and that in the prior art (MSC-FBS).
**Figure 1B** shows the percentage of cells expressing CD146 in mesenchymal stem cells obtained by continuous culture for 10 passages by the mesenchymal stem cell culture method of the present invention (MSC-AB) and that in the prior art (MSC-FBS).
**Figure 2** shows the gene expression levels of CD146, MYC, KLF4, GMNN, SOX2 and NANOG in CD146+++ strongly positive cells and CD146+ weakly positive cells in the heterogeneous stem cell population obtained by the method of the present invention.
**Figure 3A** shows the induction results of vascular endothelial cells from the heterogeneous stem cell populations cultured in the culture system of the present invention (AB) and that in the prior art (FBS).
**Figure 3B** shows the relative expression of angiogenesis-related genes in the heterogeneous stem cell populations cultured in the culture system of the present invention (AB) and that in the prior art (FBS).
**Figure 3C** shows the relative expression of angiogenesis-related genes in CD146+++ strongly positive cells and CD146+ weakly positive cells in the heterogeneous stem cell population obtained by the method of the present invention.
**Figures 4A and 4B** show that compared with human embryonic lung fibroblasts of the control group (MRC-5), the heterogeneous stem cells of the present invention can significantly increase the activation ratio of T cells.
**Figure 4C** shows that CD28 expression is up-regulated after T cells are co-cultured with the heterogeneous stem cell population obtained by the method of the present invention.
**Figure 4D** shows the expression of CD28 in CD28+ and CD28- populations sorted by magnetic bead sorting method, and the unsorted population which are co-cultured with the heterogeneous stem cell population of the present invention respectively.
**Figure 4E** shows T cell activation by CD monoclonal antibodies after CD28+, CD28-sorted by magnetic bead sorting method and unsorted populations are co-cultured with the heterogeneous stem cell population of the present invention respectively.
**Figures 4F and 4G** show that after LPS induction, the heterogeneous stem cell population of the present invention transforms into a pro-inflammatory subtype and can promote T cell activation *in vitro*; while after being induced by PolylC or IFN-γ+TNF-α (I+T), the heterogeneous stem cell population of the present invention transforms into an anti-inflammatory subtype and can inhibit T cell activation.
**Figures 4H and 4I** show that in the *in vivo* experiment using the rat acute kidney injury model, the administration of pro-inflammatory subtype of the heterogeneous stem cell population by infusion significantly increases the creatinine level in the rat urine.
Figures 5-1A to 5-1C show that the cell clustering of MSCs is significantly changed after treatment with small molecule CZ, as demonstrated with the single-cell sequencing data.

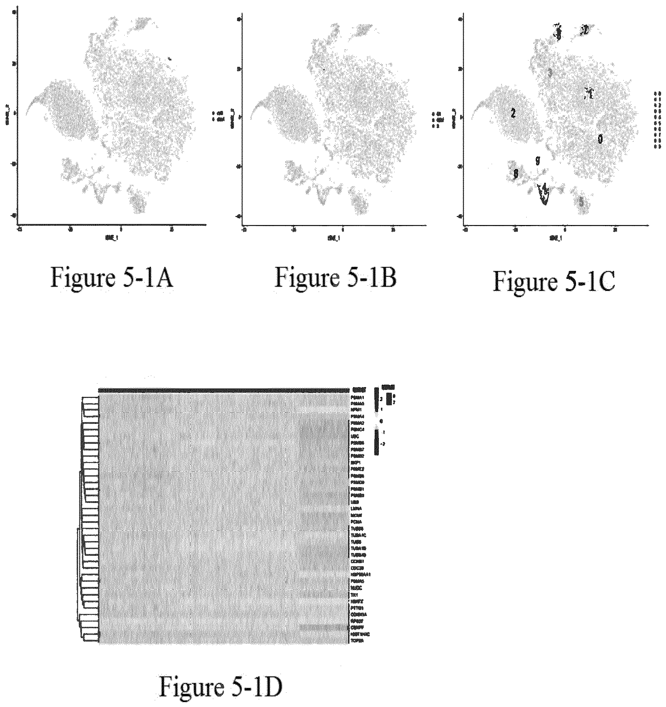

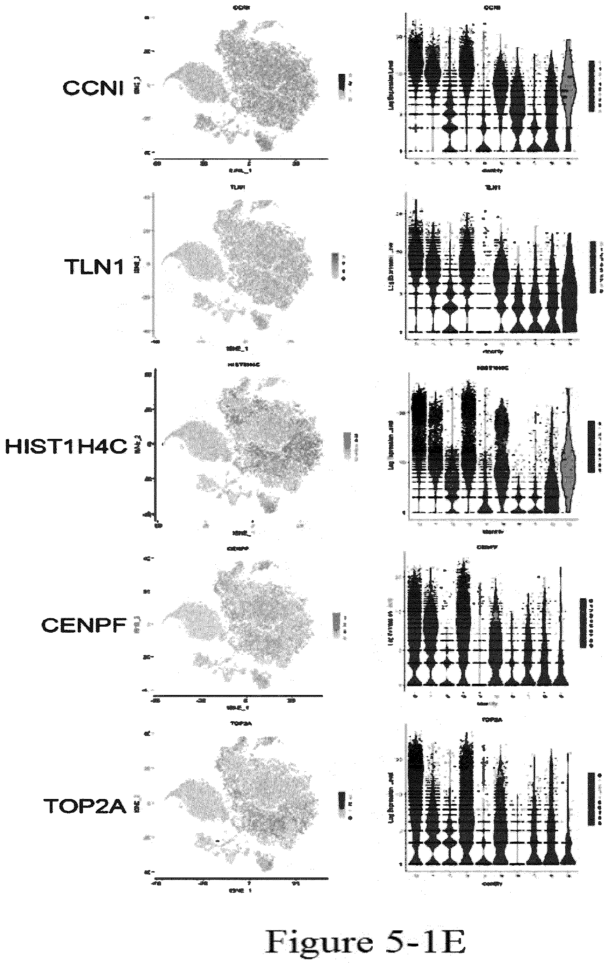

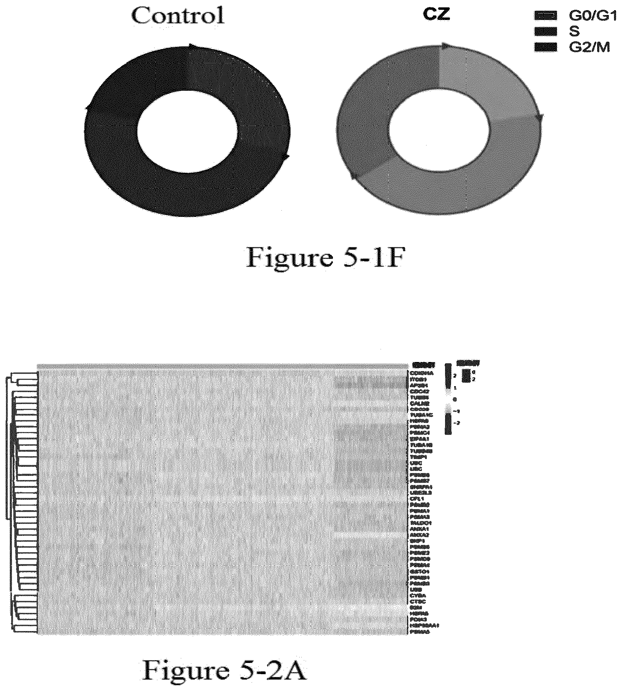

**Figure 5-1D** shows the expression of cell cycle-related genes in the cell population.
**Figure 5-1E** shows that the genes of cyclin CCNI, histone HIST1H4C, centromere protein CENPF, DNA topoisomerase TOP2A and cytoskeleton protein TLN1 are all expressed in the cell population.
**Figure 5-1F** shows that MSCs treated with small molecule CZ are more in the G2/M phase of the cell cycle, while the proportion of cells in the G0/G1 phase is reduced.

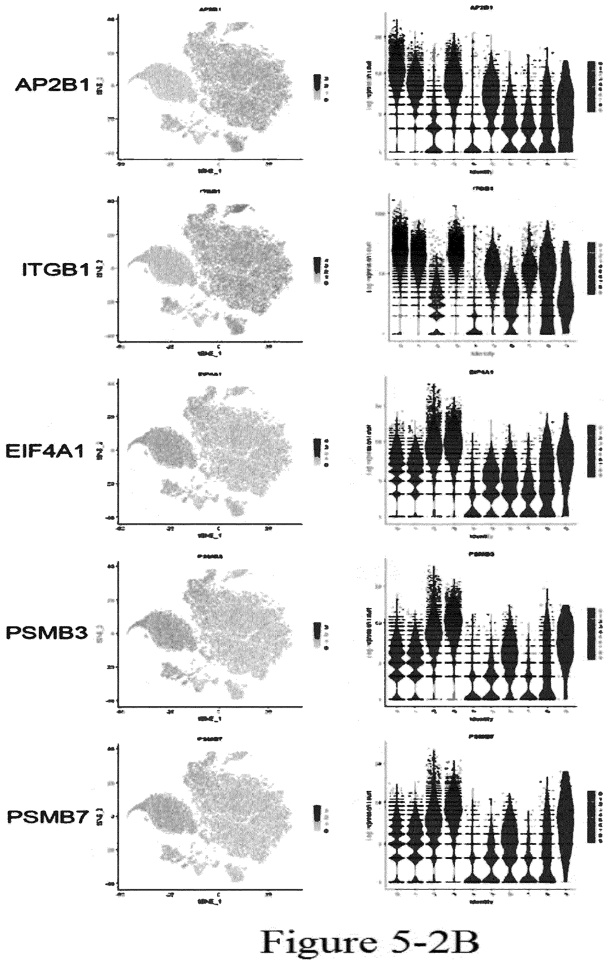

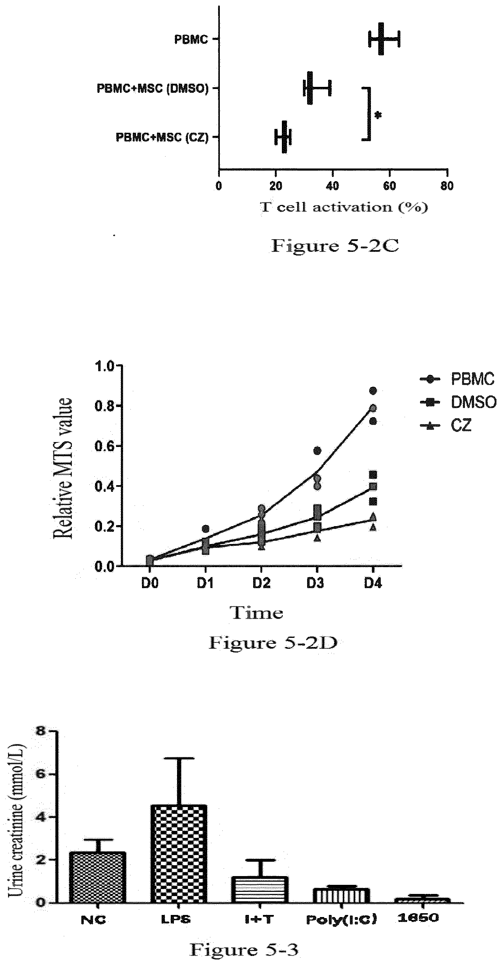

**Figure 5-2A** shows the expression of genes related to innate immunity as demonstrated with the single-cell sequencing data.
**Figure 5-2B** shows the gene expression of transporter AP2B1, integrin β1 ITGB1, EIF4A1, PSMB3 and PSMB7.
**Figure 5-2C** shows that the activation rate of PBMCs is lower after co-culture with MSCs which have been treated with small molecule CZ.
**Figure 5-2D** shows that the proliferation of PBMCs is slower after co-culture with MSCs which have been treated with small molecule CZ.
**Figure 5-3** shows the urinary creatinine level in rats with kidney injury after being treated with MSCs which have been treated in different ways.

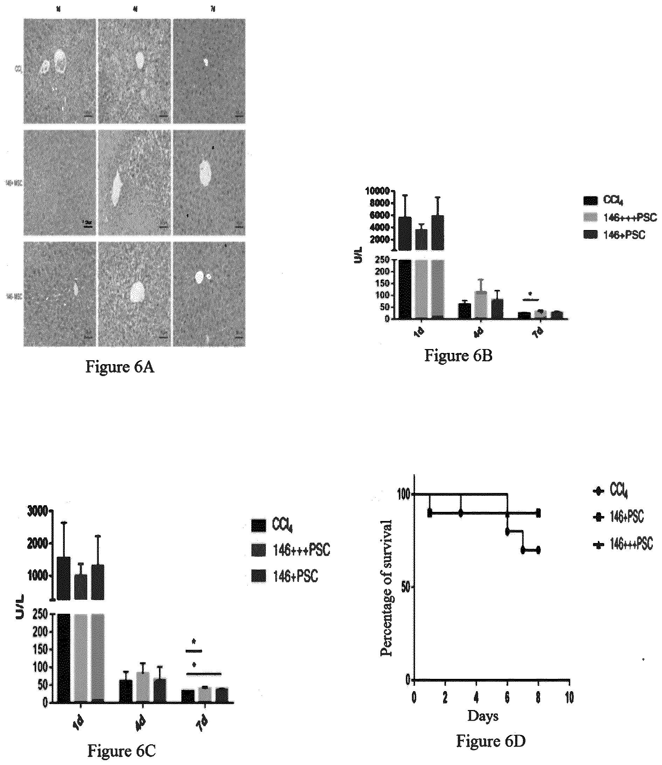

**Figure 6A** shows the inflammatory cell infiltration in C57BL/6 mice with ALI induced by the intraperitoneal injection of CCl4, on day 1, 4 or 7 after the injection of CD146+ weakly positive stem cells, CD146+++ strongly positive stem cells or PBS.
**Figure 6B** shows the ALT levels in C57BL/6 mice with ALI induced by the intraperitoneal injection of CCl4, on day 1, 4 or 7 after the injection of CD146+ weakly positive stem cells, CD146+++ strongly positive stem cells or PBS.
**Figure 6C** shows the AST levels in C57BL/6 mice with ALI induced by the intraperitoneal injection of CCl4, on day 1, 4 or 7 after the injection of CD146+ weakly positive stem cells, CD146+++ strongly positive stem cells or PBS.
**Figure 6D** shows the survival percentages of the mice.

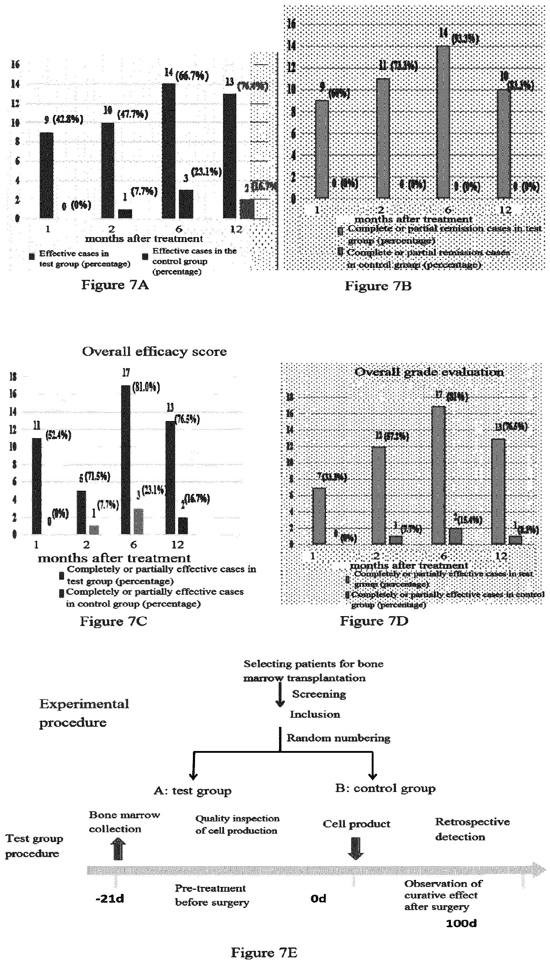

**Figures 7A to 7D** show the experimental results of the heterogeneous stem cell population of the present invention in treating cGVHD.
**Figure 7A** shows the overall efficacy score of the skin.
**Figure 7B** shows the P-ROM score of the joint.
**Figure 7C** shows the functional assessment scale (FAS) of the primary efficacy indicators during the follow-up period of 1 year. Among them, the efficacy is deemed as effective if any evaluation score of the skin, the joint and fascia, or the overall score reaches the threshold of effectiveness. The number of partially effective cases in the test group is greater than that in the control group 1, 2, 6 and 12 months after treatment, and the differences are statistically significant. The overall efficacy score of the test group is significantly better than that of the control group.
**Figure 7D** shows the FAS of other main efficacy indicators during the follow-up period of 1 year. Among them, for the overall efficacy score, the number of partially effective cases in the test group is greater than that in the control group 1, 2, 6 and 12 months after treatment, and the differences are statistically significant. The overall grade score of the test group is significantly better than that of the control group.
**Figure 7E** shows the flowchart of the experimental procedure for using the heterogeneous stem cell population of the present invention to treat cGVHD.

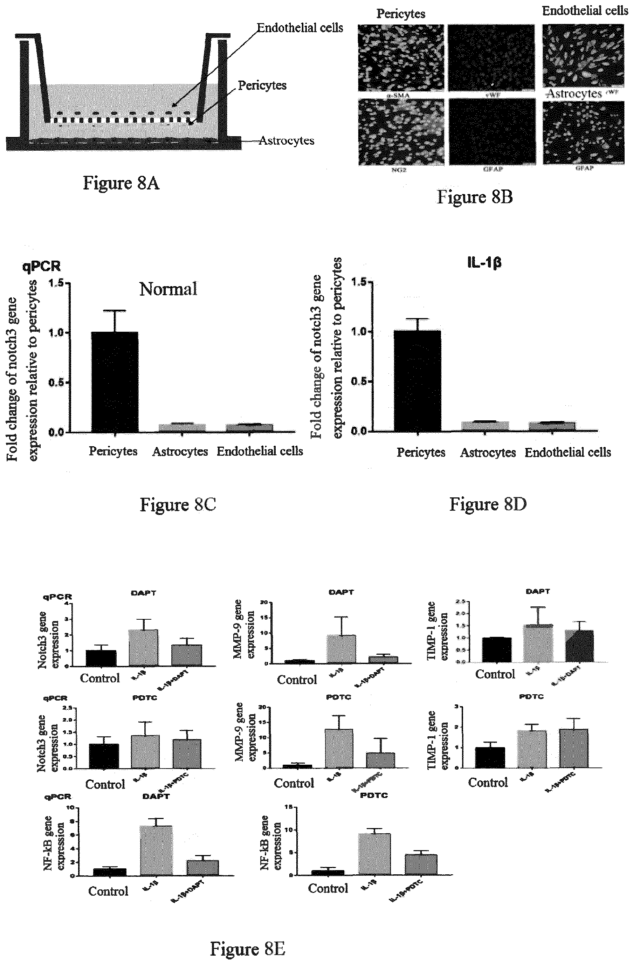

**Figure 8A** shows the schematic diagram of a model for culturing the blood-brain barrier *in vitro* by utilizing Transwell. Brain microvascular endothelial cells are cultured on the upper part, with pericytes beneath them, and astrocytes are cultured at the bottom of Transwell.
**Figure 8B** shows the identification of brain microvascular pericytes, endothelial cells and astrocytes by immunofluorescence staining. Pericytes are positive for α-SMA and NG2 expression, while negative for vWF and GFAP expression; endothelial cells are positive for vWF expression; astrocytes are positive for GFAP expression.
**Figures 8C and 8D** show the gene expression of NOTCH3in the three types of cells under the normal condition and the action of IL-1β, respectively, and the expression of MMP-9 in the three types of cells under the action of IL-1β.
**Figure 8E** shows the gene expression of NOTCH3, MMP-9, TIMP-1 and NF-κB in pericytes under the action of IL-1β with or without DAPT or PDTC, respectively; wherein, the expression changes are fold changes relative to the control.

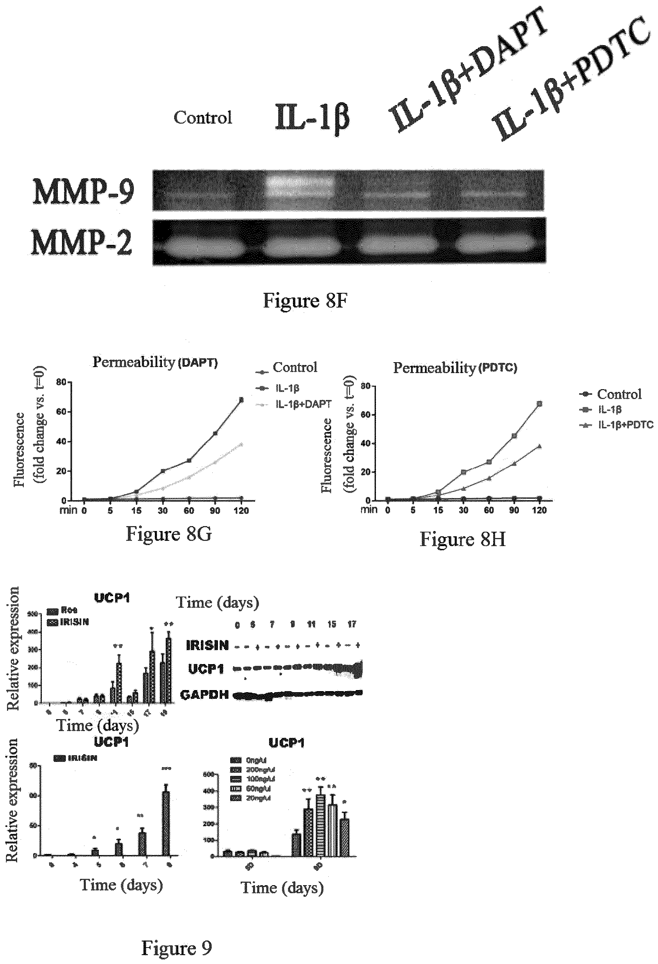

**Figure 8F** shows the changes in MMP-9 and MMP-2 activities in the control group and different treatment groups (IL-1β, IL-1β+DAPT, IL-1β+PDTC) analyzed by gelatin zymography.
**Figures 8G and 8H** show the changes in BBB permeability in the control group and different treatment groups (IL-1β, IL-1β+DAPT, IL-1β+PDTC) detected by utilizing Na-F.
**Figure 9** shows that IRISIN induces the differentiation of subpluripotent stem cells into beige adipocytes, with the expression of marker protein UCP1 detected by real-time fluorescence quantification and western blotting (P<0.05).

### DETAILED DESCRIPTION OF THE INVENTION

### Example 1 Method for isolation and in vitro culture expansion of stem cell populations

The method of the present invention includes obtaining isolated human tissues, including but not limited to: the peripheral blood, the bone marrow, the amnion, the adipose tissue, the placenta, the umbilical cord, the muscle and the skin. The tissues are digested with collagenase and undergo gradient centrifugation and filtration, followed by culture expansion in an *in vitro* culture system for up to 10 passages. The new stem cell population obtained by isolation and culture by this method are c-Myc, Gmnn, Klf4 and CD146 weakly positive; the *in vitro* culture system contains 50%-99% DMEM/F12, 0.1-30 ng/ml epidermal growth factor, 0.1-2% B27 and 0.1-10% FBS. The detailed culture steps were:
(1) Adipose MSCs were separated and extracted from the placenta, the umbilical cord, the muscle or the skin;
(2) The obtained adipose tissue was aliquoted into centrifuge tubes. A corresponding volume of PBS was added and mixed well with the tissue. Then the mixture was centrifuged at 800 rpm for 3 min and washed twice. After the washes, a corresponding volume of 0.2% collagenase was added to each tube for digestion on a shaker at 37°C for 30 min;
(3) PBS was added to terminate the digestion. The mixture was filtered with a 100 mm sieve and centrifuged at 1500 rpm for 10 min. The adipose and supernatant were discarded to obtain the cell pellet, which was then washed twice by adding PBS and centrifuged at 1500 rpm for 8 min. The cells were seeded in the manner of adding 20 ml of adipose in one T75.
(4) Passaging: After the observation of cell morphology and density, the old medium was discarded and the cells were washed twice with PBS. Then 10 ml 1× trypsin was added to digest for about half a minute, and the digestion was stopped with a few drops of serum. The mixture was collected and centrifuged at 1200 rpm for 5 min, and the supernatant was discarded. The cells were re-suspended in a corresponding volume of new medium and cultured in petri dishes.

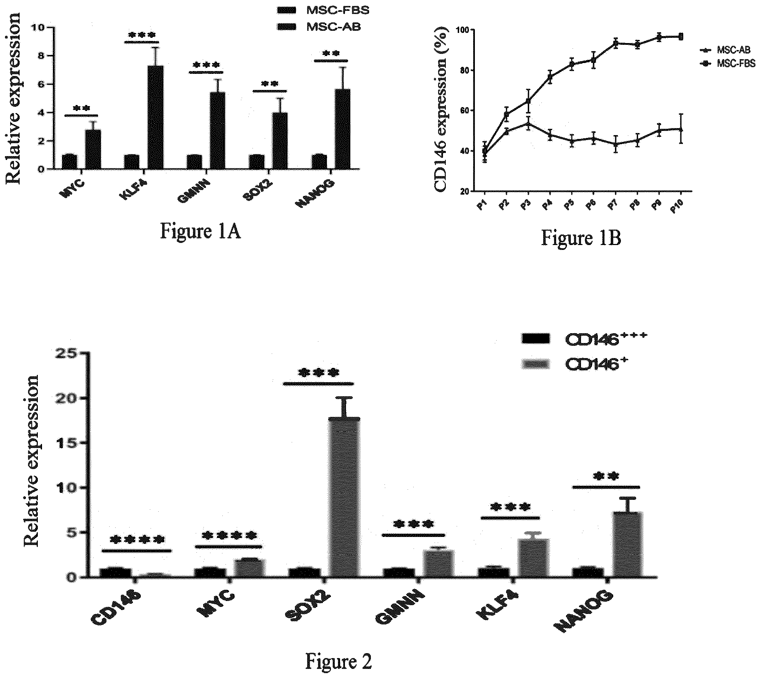

Through the method for culturing heterogeneous stem cell populations established by the inventor, it was found that by using the culture system of our laboratory, higher levels of *in vitro* expression of stemness-related genes MYC, KLF4, GMNN, SOX2 and NANOG were achieved compared with the stem cell population cultured in conventional heterogeneous stem cell population culture system (FBS system) (see Figure 1A). The CD146 positive rate in the heterogeneous stem cell population cultured in FBS culture system was significantly increased, reaching more than 90% after 7 passages, showing a phenotype similar to the heterogeneous stem cell populations, whereas in the heterogeneous stem cell population cultured using our culture system, the positive rate of CD146 remained at 50% (Figure 1B).

Phenotype detection of cells by monoclonal antibodies:
The cells were collected, counted and then re-suspended at the corresponding concentrations. Then corresponding amounts of antibodies for detection were added to and mixed well with the cells. The mixture was incubated at 4°C for 30 min. Then PBS was added to wash the cells twice. The cells were centrifuged at 1000 rpm for 5 min, the supernatant was discarded, a corresponding volume of PBS was added to re-suspend the cells, followed by tests on the instrument.

### Example 2 Sorting of the heterogeneous stem cell population obtained by the method of the present invention

Next, using the magnetic bead sorting method, the CD146+++ (50% to 100% expression rate of the cell surface marker) and the CD146+ (1% to 50% expression rate of the cell surface marker, excluding 50%) cell populations were separated from the new stem cells cultured in our culture system for comparison.

The detailed sorting steps were described as follows: CD146+ positive selection was conducted to sort umbilical cord-derived mesenchymal stem cells with immunomagnetic beads by using the Vario-MACS system:
(1) The cell pellet was collected and the supernatant was discarded.
(2) The cell pellet was re-suspended in a buffer (containing D-Hanks, 0. 5% BSA, 2 mMol EDTA) at a ratio of 10⁷ cells per 60 µl of buffer. FcR and CD146+ magnetic beads were added to the cell suspension at a ratio of 20 µl per 10⁷ cells, mixed well by pipetting and incubated with the cells for 15 min in a refrigerator at 4°C.
(3) The cells were washed at a ratio of 10⁷ cells per 1 ml of buffer. The precipitate was collected.
(4) The cells were re-suspended at a ratio of 10⁷ cells per 500 µl buffer.
(5) The LP sorting column was put into the magnetic field of the Vario-MACS system, and 3 ml buffer was added to wash the sorting column 3 times. After the buffer completely flowed out of the sorting column, the cell suspension was added.

The column was washed with an appropriate amount of buffer. After all the liquid flowed out, the LP sorting column was removed from the magnetic field of the MACS system and was placed on a collection tube. 5 ml buffer was added and a piston was inserted to quickly flush out the cells, obtaining the CD146+++ cell subset labeled with CD146+ magnetic beads. It was found that the expression levels of the above-mentioned stemness genes in CD146+ cells were significantly higher than those in CD146+++ cells (see Figure 2). These results suggested that as for the heterogeneous stem cell populations obtained by the method of the present invention, the stemness of CD146+++ cells was lower than that of CD146+ cells, and the former were a stem cell subpopulation located downstream of the latter in the differentiation lineage. Meanwhile, the heterogeneous stem cell population cultured in the culture system described herein could maintain a high proportion of CD146+ cells after *in vitro* passaging. The culture system described herein is called the stemness maintenance system of heterogeneous stem cell populations.

### Example 3 The induction experiment of vascular endothelium in vitro

The experimental steps were detailed as follows:
(1) Matrigol (BD, low growth factor, 354230) was pre-chilled and melted in advance and aliquoted into 96-well plates (This step could also be performed directly on the back of a freezing plate). Melting could be started one hour in advance for small volumes. Otherwise, overnight melting was required for bigger volumes.
(2) The gel was vertically added at 40-55 µl per well while carefully preventing any bubbles. The plate was equilibrated at room temperature for 10 minutes and was then kept still at 37°C for half an hour.
(3) The cells were prepared during the settling period of the gel. (The gel could also be prepared one week in advance.)
(4) The cell suspension was added at 150 µl per well.

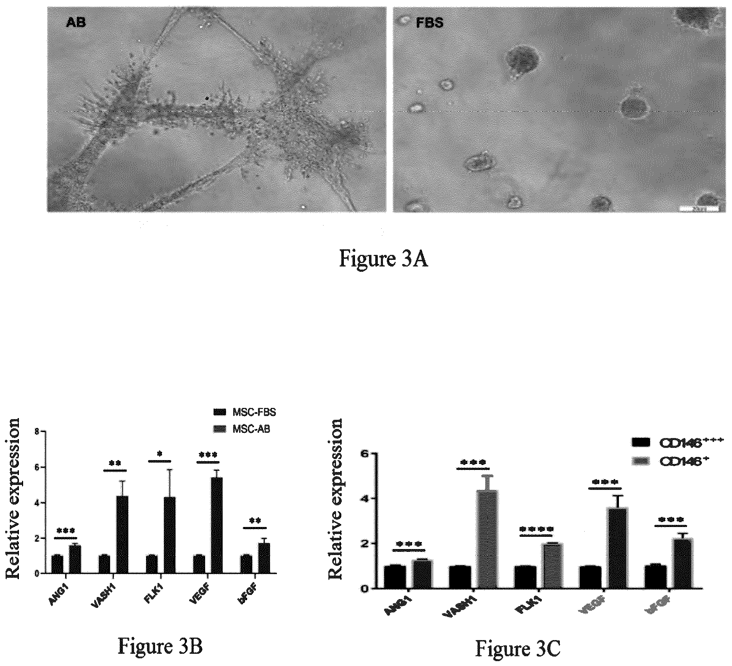

The heterogeneous stem cell population cultured in the stemness maintenance system of the present invention could smoothly induce the vascular endothelium generated to form a network, while the cells of the MSC culture system in the prior art could not (see Figure 3A). Further examination of Ang-1, VASH1, FLK, VEGF and bFGF showed that the heterogeneous stem cell population cultured in the stemness maintenance system of the present invention highly expressed these angiogenesis-related genes during the induction process. Similar results were also obtained by comparing the sorted CD146+++ and CD146+ cells (see Figure 3B and Figure 3C).

As shown by the results above, the heterogeneous stem cell population obtained from the culture system of the present invention (AB) has stronger stemness and stronger vascular endothelium genesis ability.

### Example 4 The heterogeneous stem cell population of the present invention has

### relatively strong immunomodulatory ability

The experimental methods and steps were detailed as follows:
(1) The stem cells were sub-cultured for 3-5 passages in the AB liquid culture system.
(2) After 7 days of co-culture with resting peripheral blood mononuclear cells (PBMCs) *in vitro* (1640+10% FBS), the stem cells were removed and the T cells were activated with CD3 antibody for 72 h.
(3) Compared with the human embryonic lung fibroblasts (MRC-5) co-culture control group, our stem cell co-culture group could significantly increase CD28 expression in T cells and T cell activation ratio.

It has been reported that MSCs have a strong immunomodulatory effect, and many experiments have proved that MSCs can inhibit the activation and proliferation of T cells *in vitro.* The heterogeneous stem cell population cultured in the stemness maintenance system of the present invention has a strong immunomodulatory effect.

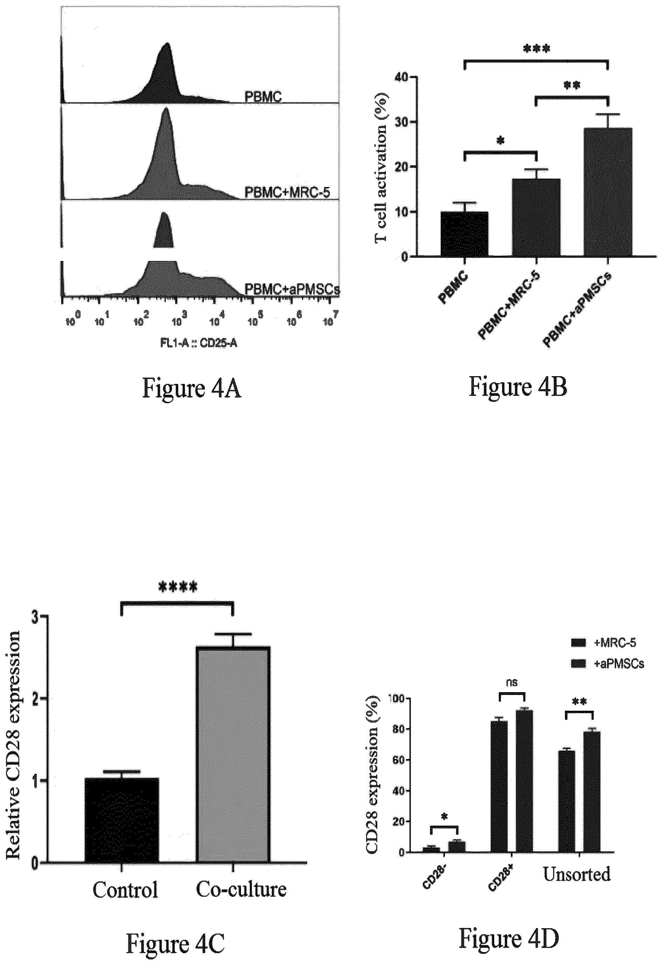

First, the heterogeneous stem cell population obtained by the method of the present invention was co-cultured with resting PBMCs for 7 days *in vitro.* Then the stem cells were removed and the T cells were activated by CD3/CD28 antibody co-stimulation. It was found that compared with human embryonic lung fibroblast (MRC-5) control group, the heterogeneous stem cells of the present invention could significantly increase T cell activation ratio (see Figure 4A and Figure 4B), while ordinary MSCs usually inhibit T cell activation.

The results suggested that in the process of co-culturing the heterogeneous stem cell population of the present invention with T cells, the stem cell population may enhance the activation potential of T cells. In order to prove this possibility, the gene expression of CD3 and CD28 was tested in the co-stimulatory activation pathway before and after co-culture of T cells with the heterogeneous stem cell population of the present invention. As expected, it was found that the CD28 gene was significantly up-regulated after co-culture (see Figure 4C).

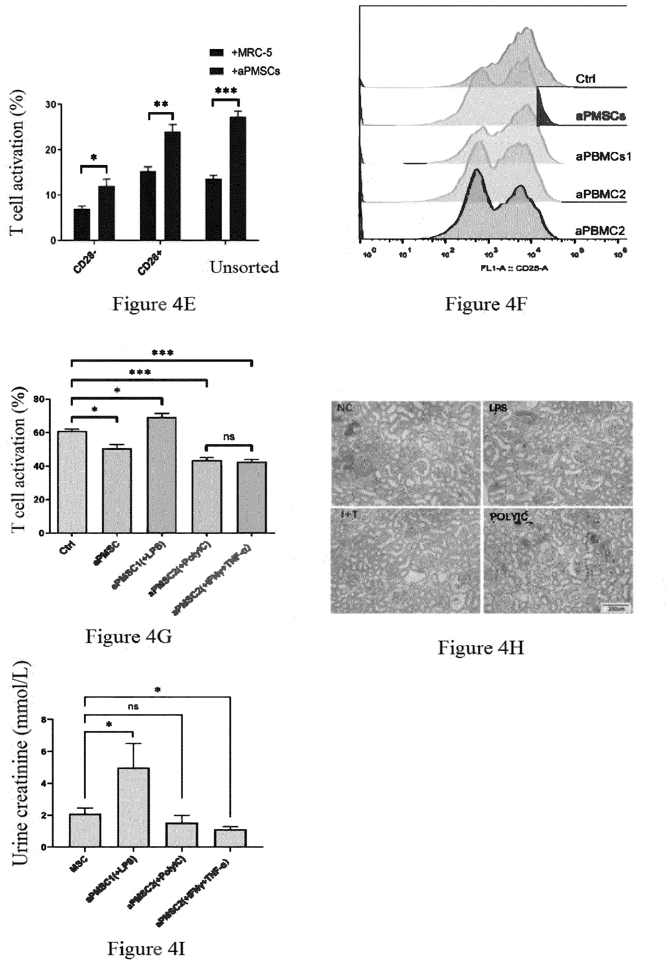

In order to further demonstrate the source of up-regulation of CD28 molecule, a magnetic bead sorting method was used to separate CD28+/- T cells, which were co-cultured with the heterogeneous stem cell population of the present invention respectively. Compared with the control group, significant up-regulation of CD28 molecules in the CD28-T cell population and the unsorted T cell population was found (see Figure 4D). T cell populations co-cultured after sorting were activated by CD monoclonal antibodies (to remove the effect of CD28 antibodies), and it was found that after co-culture with the heterogeneous stem cell population of the present invention, the activation rate of each T cell population was all significantly increased (see Figure 4E).

In addition, the heterogeneous stem cell population of the present invention also has immunoplasticity similar to ordinary MSCs. The heterogeneous stem cell population of the present invention transformed into a pro-inflammatory subtype by LPS induction, which could promote T cell activation *in vitro*; while the heterogeneous stem cell population of the present invention transformed into an anti-inflammatory subtype by PolylC or IFN-γ+TNF-α(I+T) induction, which could inhibit T cell activation (see Figure 4F and Figure 4G).

In the *in vivo* experiments in rat acute kidney injury model, it was found that the infusion of the pro-inflammatory subtype of the heterogeneous stem cell population into rats would significantly increase the urine creatinine level, and pathological sections showed increased renal inflammatory cell infiltration and increased fibrinoid necrosis. Meanwhile, the infusion of the anti-inflammatory subtype into rats greatly reduced inflammatory cell infiltration and reduced mesangial cell lysis and proliferation. Among them, the I+T group mainly showed reduction of inflammatory cell infiltration, and the PolylC group mainly showed reduction of cell proliferation (see Figure 4H and Figure 4I).

Conclusion: The new stem cells obtained by the method of the present invention are a mixed heterogeneous stem cell population, which can maintain the pluripotency in the stemness maintenance culture system of the present invention for at least 10 passages. This is an important feature that distinguishes them from ordinary MSCs, and enables the heterogeneous stem cell population of the present invention to be induced to differentiate into downstream functional cells such as vascular endothelial cells, an important indicator cell population in vascular aging.

CD146 (MCAM) is a cell adhesion molecule that is commonly used as a marker for endothelial cells and pericytes. Recent studies have also shown that CD146 is a receptor for nerve growth factor (netrin) and a co-receptor for vascular endothelial growth factor receptor 2 (VEGFR2). The experimental results confirmed that there is a close correlation between the CD146 molecule and the stemness of the heterogeneous stem cell population of the present invention. Whether CD146 can become a potential marker for new stem cells requires further research in the future.

CD28 is an immune co-activation molecule on the surface of T cells. Almost all T cells are CD28+ at birth. As the body is exposed to antigens in the process of growth and aging, T cells gradually lose the CD28 phenotype and the ability to activate. Therefore, CD28 becomes a molecule that indicates the aging of the immune system. The new stem cell population of the present invention can significantly increase the proportion of T cell population expressing CD28 after co-culture with PBMCs. Therefore, it is believed that new stem cells can enhance the activation potential and immune function of T cells, especially for people over 65 years old, among whom 50-60% of CD8+ T cells and 5-10% of CD4+ T cells lack CD28 molecules. This discovery makes the treatment of immune system aging with new stem cells a very promising cell therapy.

### Example 5 Small molecule CZ can induce the heterogeneous stem cell population of the

### present invention into an anti-inflammatory stem cell population

The product name of small molecule CZ is chlorzoxazone. Catalog number: TCZ; CAS number: 95-25-0; its molecular formula is C₇H₄ClNO₂; its molecular weight is 169.57. Storage: 2 years in solvent at -80°C; 3 years in powder at -20°C.

Chlorzoxazone is a centrally acting muscle relaxant used to treat muscle cramps and the resulting pain or discomfort. It acts on the spinal cord by inhibiting reflexes. Chlorzoxazone is currently used as a marker substrate in *in vitro*/*in vivo* studies to quantify cytochrome P4502E1 (CYP2E) activity in human body.

Its physical properties:
Boiling point: 181°C ~192°C
Melting point: N/A
Solubility: Dimethyl formamide (DMF), 34 mg/ml (200.5 mM)
Dimethyl sulfoxide (DMSO), 34 mg/ml (200.5 mM) Water

This research is intended for diagnostic or therapeutic purposes only.

The product data sheet lists the information for product storage and handling, and the Targetmol product is stable over long periods of time under the recommended storage conditions. The described products may be shipped under different conditions, because many products remain stable for short periods of time at higher or even room temperatures. It is ensured that the products are shipped under conditions that maintains the quality of the reagents. After receiving the product, please follow the storage recommendations in the product data sheet for further operations.

The inventor found that the heterogeneous stem cell population could differentiate into anti-inflammatory MSC2 (a subtype of stem cell population) under the induction of small molecule CZ. Combining the results of *in vitro* experiments, the transformation of heterogeneous stem cell population of the present invention into anti-inflammatory MSC2 was further studied by single cell sequencing. After the treatment with CZ, the cells entered a state of anti-inflammatory function activation similar to MSC2, which would be used for clinical treatment of autoimmune diseases.

### Small molecule CZ can increase the proliferation ability of MSCs

Using the single-cell sequencing method, it was found that after 48 hours of pretreatment with small molecule CZ, the cell clustering of MSCs changed significantly (Figure 5-1A). The expression of genes related to cell cycle showed that MSCs treated with small molecule CZ expressed genes of the S phase and G2/M phase more (Figure 5-1B). After careful cluster analysis of the control group and the small molecule CZ treatment group, it was found that cell group 2 (cluster2) was the cell population with the largest change in them. Cell group 2 completely disappeared in the samples after CZ treatment, and it only expressed genes of the G1 phase and S phase, but not genes of the G2/M-phase (Figure 5-1A, Figure 5-1B, Figure 5-1C). Therefore, a careful analysis of genes related to cell cycle in cell population 2 was performed. It was found that the expression levels of genes related to cell cycle in cell population 2 had significant difference from those in other cell populations (Figure 5-1D). Among them, the genes of cyclin CCNI, histone HIST1H4C, centromere protein CENPF, DNA topoisomerase TOP2A and cytoskeleton protein TLN1 were all relatively lowly expressed in cell population 2 while highly expressed in other cell populations (Figure 5-1E).

*In vitro* experiments also showed that MSCs treated with small molecule CZ were more in the G2/M phase of the cell cycle, while the proportion of cells in the G0/G1 phase was reduced (Figure 5-1F).

### Small molecule CZ can increase the immunomodulatory ability of MSCs

By further analyzing the single-cell sequencing data, it was found that cell group 2 is also significantly different from other cell populations in the expression of genes related to innate immunity (Figure 5-2A). Among proteins with the most significant differences, it was found that the genes of transporter AP2B1 and integrin β1 ITGB1 were lowly expressed in cell population 2. Among them, the former can participate in TGFβ receptor-mediated endocytosis, while the latter participates in IL1β receptor-mediated IL1β signaling pathway, and plays an important role in immunity against microbial infection. On the contrary, the genes of PSMB3 and PSMB7, involved in the formation of the 20s protease complex, were highly expressed in cell group 2, indicating slower proteolysis of ubiquitination in cells other than cell group 2, and thus resulting in generally stronger protein functions (Figure 5-2B).

*In vitro* experiments also showed that after co-culture with MSCs which have been treated with small molecule CZ, PBMCs had lower activation rate (Figure 5-2C) and slower proliferation (Figure 5-2D). Thus, small molecule CZ can increase the immunosuppressive ability of MSCs.

Conclusion: After analyzing the single-cell data in multiple aspects, it is believed that cells of group 2 are a relatively resting cell population which are in a functionally primitive state without being activated by the small molecule. Whereas, after treatment with the small molecule CZ, the new stem cell population enters a state of functional activation, the cell cycle is accelerated, the proliferation rate is increased, and the expression of genes related to immunomodulation functions also changes accordingly.

The immunosuppressive function of MSCs has been widely known, and MSCs have successfully entered the clinical stage for the treatment of autoimmune diseases such as GvHD, etc. However, in the course of treatment, for a small number of cases, the treatment is not as effective as expected or even has the opposite effect. MSCs are a heterogeneous cell population. It is believed that if the proportion of cells in the MSC population that can effectively suppress the immune response is not enough, the *in vivo* curative effect will be greatly reduced.

At present, a theory has been proposed that MSCs are divided into pro-inflammatory MSC1 and anti-inflammatory MSC2. It is known that LPS can induce pro-inflammatory MSC1, and IFN-γ+TNF-α, poly(I:C) can induce MSCs to transform into anti-inflammatory MSC2. However, as MSCs exhibited significantly enhanced immunogenicity after treatment in this way, they cannot be widely used in clinical treatment. Thus, it is hoped to find a clinical-grade small molecule that can enhance the immunosuppressive function of MSCs or increase the proportion of MSC2 in MSC population, while not significantly changing the immunogenicity of MSCs.

With big data mining and functional screening, a small molecule compound with the code name CZ was identified, which is a clinical drug approved by the FDA. In the rat model of acute kidney injury, it was found that infusion of the pro-inflammatory subtype MSC1 (LPS treatment group) would significantly increase the level of urinary creatinine; while infusion of the anti-inflammatory subtype MSC2 (I + T, poly(I:C), CZ group) significantly reduced the level of urine creatinine, and the effect of small molecule CZ was the most significant (Figure 5-3). In the pathological tissue sections, pro-inflammatory MSC1 increased renal inflammatory cell infiltration and aggravated glomerular fibrinoid necrosis; while anti-inflammatory MSC2 greatly reduced inflammatory cell infiltration and reduced mesangial cell lysis and proliferation.

### The screening process of the small molecule CZ:

1. Big data mining of the interacting proteins of IDO, a key molecule in human MSC immunomodulation;
2. Screening the function of the obtained proteins, thereby obtaining CZ molecule that can enhance the immunosuppressive function of MSCs.

### Example 6 The heterogeneous stem cell population of the present invention and tissue damage repair

In this example, the different curative effects of the heterogeneous stem cell population of the present invention in acute liver injury (ALI) were investigated.

C57BL/6 mice were intraperitoneally injected with CCl4 to induce ALI. 6 hours later, ALI mice were injected with 5×10⁵ CD146+ stem cells, CD146+++ stem cells or PBS (placebo treatment group). On day 1, there was more inflammatory cell infiltration in the control group, and all three groups had slight balloon-like changes. On day 4, the control group had extensive balloon-like changes and massive inflammatory cell infiltration, while large necrotic lesions and extensive globular changes were observed in the CD146+++ stem cell transplantation group. On day 7, the three groups returned to a state of normal liver histology (see Figure 6A), and the CD146+++ stem cell transplantation group had significantly higher levels of serum liver enzymes alanine aminotransferase (ALT, p = 0.027) and aspartate aminotransferase (AST, p = 0.012) (see Figure 6B and Figure 6C). In addition, the numbers of CD146+++ and CD146+ stem cells in the liver were the highest on day 4, and the number of CD146+++ stem cells in the liver was lower than that of CD146+ stem cells.

The survival rate of the CD146+ stem cell treatment group was significantly higher than that of the placebo treatment group (82% vs. 54.5%) (see Figure 6D). The peak levels of ALT and AST in the PSC treatment group were significantly lower than those in the placebo treatment group (P < 0.01). Therefore, PSC administration significantly reduced acute liver injury induced by CCl4.

### Example 7 The curative effect of the heterogeneous stem cell population of the present invention on cGVHD

From January 2012 to October 2015, a total of 35 eligible patients were recruited. The patients were divided into two groups and received first-line immunosuppressive therapy. The test group received an additional 4 doses of intramedullary infusion of the heterogeneous stem cell population of the present invention, wherein the procedure of the clinical trial was shown in Figure 7E.

The results showed that infusion of the heterogeneous stem cell population of the present invention significantly improved the clinical results of refractory ScGVHD, enhancing the overall treatment response and alleviating the overall severity of cGVHD symptoms including those of skin, joints and fascia (P < 0.05) (see Figure 7A - Figure 7D). The overall response rate (ORR) of the test group reached a peak of 82.1% at 6 months, while the ORR of the control group was only 23.1%. No early- or late-stage safety issues related to MSC infusion were observed.

According to the data above, the joints and fascia seem to be more sensitive to PSC treatment, with a 90% RR at 6 months, while only half of the patients had skin response. To determine the prognostic factors of skin response to PSC treatment, baseline demographic and clinical characteristics (including gender, age, disease, donor, cGVHD duration, baseline NIH skin score, KPS and PSCs dose) were compared between skin responders and non-skin responders. The skin responders and non-responders only had a marginally significant difference in the donor type (P = 0.077). At 6 months, the skin response rate of patients receiving transplantation from matched sibling donors was 72.7%, while the response rate of patients receiving transplantation from half matched donors was only 22.2%.

Flow cytometry was performed to measure the T lymphocyte subpopulations at baseline and during the 1-year follow-up period between the two arms. Both Treg percentage and Th1/Th2 ratio increased after PSC injection, reached the peak after 2 PSC injections, and gradually decreased during the follow-up period. At each evaluation point, no difference in Treg percentage and Th1/Th2 ratio was observed between the two groups (P > 0.05).

A total of 112 PSC intramedullary infusions were administered to 28 patients. All infusions were well tolerated. No acute infusion-related reactions and PSC infusion-related adverse events were observed. During the follow-up period, a total of 6 deaths were observed among the registered patients.

### Example 8 Use of the heterogeneous stem cell population of the present invention in the intervention of blood-brain barrier drugs

The blood-brain barrier consists of brain microvascular endothelial cells, pericytes and astrocytes. Pericytes play a very important role in the blood-brain barrier. At the same time, they belong to the new stem cell population/pericyte population which contains the main functional cells of the sanjiao structure in traditional Chinese medicine. The sanjiao structure is associated with the viscera, organs and tissues of the whole body, achieving the regulation of human meridian function through the circulation of qi and blood, the conduction of reaction, and the coordination with each other. The sanjiao organ/mesenchymal tissue system is mainly responsible for stem cell proliferation and differentiation to multiple tissues, as well as the regenerative repair and functional reconstruction of organs and tissues. It participates in human immune surveillance, immune response and the regulation of complex immune network. It plays a systemic regulatory role in brain diseases and nerve repair, the modulation of interstitial hormones, endocrine regulation and tissue metabolism.

Imaging findings suggest that in white matter lesions, the blood-brain barrier is damaged, wherein inflammation is one of the reasons involved. The pathogenesis of brain injury in small vessel disease lies in the blood-brain barrier damage, which is caused by local inflammation. The involvement of inflammation in blood-brain barrier damage has not been fully elucidated.

The heterogeneous stem cell population obtained by the method of the present invention was used to induce directed differentiation into brain microvascular endothelial cells, pericytes and astrocytes, thereby constructing a blood-brain barrier.

The method and experimental steps for inducing differentiation and the establishment of the blood-brain barrier were described as follows:
Interleukin 1β (IL-1β) were added to Transwell *in vitro* blood-brain barrier model, and cultured human brain microvascular endothelial cells (HBMVECs), human brain pericytes (HBPs) and human brain astrocytes (HBAs) *in vitro.* HBMVECs, HBPs and HBAs were verified by immunofluorescence staining. Among them, HBMVEC was stained with the antibody against vWF; HBP was stained with the antibodies against α-SMA and NG2, and vWF and GFAP antibodies; and HBA was stained with GFAP antibody (Figure 8B). The q-PCR results suggested that NOTCH3 was expressed in HBPs and not in HBMVECs and HBAs under normal conditions (Figure 8C). After being co-cultured with IL-β for 24 hours, the expression of NOTCH3 was observed in the HBP group, but not in the HBMVEC and HBA groups (Figure 8D). HBPs were treated with IL-1β for 30 minutes followed by DAPT treatment for 24 hours, and subjected to q-PCR quantification. It could be seen from Figure 8E that, compared to the control group, the gene expression of NOTCH3 in the IL-1β group was increased; compared to the IL-1β group, the gene expression of NOTCH3 in the IL-1β+DAPT group was reduced. Meanwhile, the gene expression of MMP-9 was similar to that of NOTCH3, while the gene expression of TIMP-1 among the three groups did not change much. For the gene of NF-κB p65, its expression in the IL-1β group was significantly increased compared to the control group, while compared to the IL-1β group, its expression in the IL-1β+DAPT group was significantly reduced. After the treatment with IL-1β for 30 minutes and PDTC for 24 hours, the IL-1β group of HBPs had a significant increase in Notch3 expression compared to the control group, while the IL-1β+ PDTC group did not change much in Notch3 expression compared to the IL-1β group. At the same time, compared to the control group, gene expression of MMP-9 in the IL-1β group was significantly increased, and gene expression of MMP-9 in the IL-1β+DAPT group was significantly lower than that in the IL-1β group. The gene expression of TIMP did not change significantly. Additionally, the gene expression shifts of NF-κB p65 in the PDTC-treated group were similar to those in the DAPT-treated group (see Figure 8E).

Figure 8A showed the schematic diagram of a model for culturing the blood-brain barrier *in vitro* by utilizing Transwell. Brain microvascular endothelial cells were cultured on the upper part, with pericytes beneath them, and astrocytes were cultured at the bottom of Transwell;

Figure 8B showed the identification of brain microvascular pericytes, endothelial cells and astrocytes by immunofluorescence staining. Pericytes were positive for α-SMA and NG2 expression, while negative for vWF and GFAP expression; endothelial cells were positive for vWF expression; astrocytes were positive for GFAP expression;

Figure 8C and Figure 8D respectively showed the gene expression of NOTCH3 and MMP-9 in the three types of cells under the normal condition and the action of IL-1β, respectively;

Figure 8E showed the gene expression of NOTCH3, MMP-9, TIMP-1 and NF-κB in pericytes under the action of IL-1β with or without DAPT or PDTC, respectively; wherein, the expression changes were fold changes relative to the control;

Figure 8F showed the changes in MMP-9 and MMP-2 activities in the control group and different treatment groups (IL-1β, IL-1β+DAPT, IL-1β+PDTC) analyzed by gelatin zymography analysis;

Figure 8G and Figure 8H showed the changes in BBB permeability in the control group and different treatment groups (IL-1β, IL-1β+DAPT, IL-1β+PDTC) detected by utilizing Na-F.

### Example 9 Use of the heterogeneous stem cell population of the present invention in regulating adipocyte tissue metabolism

In recent years, a new hormone IRISIN has been discovered, which is a muscle factor secreted after exercise. It is released from the cleavage of its precursor - fibronectin III domain containing 5 (Fndc5), and is involved in the function of the muscles, the cardiovascular system, the nerves and energy metabolism. In the process of energy regulation, IRISIN is a potential regulator in sugar metabolism. In this study, the heterogeneous stem cell population of the present invention was used as seed cells, and used IRISIN to induce them to differentiate into beige adipocytes (see Figure 9).

The induction method and steps were detailed as follows:
The adipose tissue was washed twice with D-Hanks' solution containing dual antibiotics (penicillin, streptomycin), and centrifuged at 800 rpm for 3 min. The lower layer of the liquid after washing was removed with a pipette and the adipose tissue was transferred to a new centrifugation tube of 50 ml. 0.2% collagenase P was added for digestion. The tube was incubated in a shaker at a constant temperature of 37°C for 30 min. An appropriate amount of D-Hanks' solution was added to the digested adipose tissue, which was then filtered with a 100 µm cell sieve to remove undigested tissue. The mixture was centrifuged at 1500 rpm for 10 min. The upper layer of grease was removed with a pipette, then the supernatant was discarded and the cell pellet was re-suspended in D-Hanks' solution and washed once. The mixture was centrifuged at 1500 rpm for 10 min. The supernatant was discarded and 12 ml of hAD-MSC working solution containing appropriate amounts of dual antibiotics was used to re-suspend 2×10⁶ cells for seeding into a T75 culture flask, and the cells were cultured in a cell incubator at a constant temperature of 37°C, and with 5% CO₂ and saturated humidity. 48 hours after seeding the primary cells, the upper layer containing non-adherent cells was removed. The culture was maintained with complete medium change every 2-3 days. When the cells reached 80% confluence, they can be sub-cultured or cryopreserved for preservation.

The new stem cell population obtained by the method of the present invention is novel not only in terms of differentiation potential, but also in respect of signal susceptibility, capable of receiving different types of signals, transforming into different subtypes, and enhancing functions in certain aspects. For example, under the stimulation of different immune microenvironments, it can be transformed into pro-inflammatory or anti-inflammatory functional subclasses. Under culture conditions with high concentrations of FBS, it transforms into a CD146+++ subgroup, which has enhanced T cell regulatory function, enhanced osteogenic differentiation ability, reduced adipogenic differentiation ability, while almost losing its angiogenic ability; while under culture conditions with low concentrations of FBS, it transforms into a CD146+ subgroup, which shows decreased immunity, increased adipogenic and angiogenic abilities, and reduced osteogenic ability. Under the stimulation of cold, exercise or emergency signals, it receives the signal of irisin secreted from muscles and transforms into precursor cells that are prone to differentiate into beige adipose.

## Claims

1. A heterogeneous stem cell population, **characterized in that** the stem cells in the heterogeneous stem cell population express stemness genes MYC, KLF4, GMNN, SOX2 and NANOG, and in the heterogeneous stem cell population, the ratio of stem cells expressing CD146 is 1-100%, preferably, the ratio of stem cells expressing CD146 is 1-50%;
optionally, wherein the expression levels of the stemness genes MYC, KLF4, GMNN, SOX2 and NANOG in stem cells with CD146+ (weakly positive) expression are significantly higher than those of the stem cells with CD146+++ (strongly positive) expression.

2. The heterogeneous stem cell population according to claim 1, **characterized in that** after subculture of the heterogeneous stem cell population in a culture medium containing 50%-99% DMEM/F12, 0.1-30 ng/ml epidermal growth factor, 0.1-2% B27 and 0.1-10% FBS for 1, 2, 3, 4, 5, 6, 7, 8, 9, 10 or more than 10 passages, the ratio of stem cells expressing CD146 is 1-100%, preferably, the ratio of stem cells expressing CD146 is 1-50%.

3. The heterogeneous stem cell population according to claim 1, **characterized in that** after subculture of the heterogeneous stem cell population in a culture medium containing 0.1-10% FBS for 1, 2, 3, 4, 5, 6, 7, 8, 9, 10 or more than 10 passages, the ratio of stem cells expressing CD146 is 50-100%, preferably, the ratio of stem cells expressing CD146 is 55-100%.

4. A method for inducing the heterogeneous stem cell population according to any one of claims 1-3 to differentiate into reticular vascular endothelial cells *in vitro,* including inducing the heterogeneous stem cell population according to any one of claims 1-4 to differentiate into reticular vascular endothelial cells in a culture medium containing 50%-99% DMEM/F12, 0.1-30 ng/ml epidermal growth factor, 0.1-2% B27 and 0.1-10% FBS.

5. A method for regulating T cell activation *in vitro,* including the step of co-culturing the heterogeneous stem cell population according to any one of claims 1-3 and isolated PBMCs in a culture medium containing LPS, thereby promoting T cell activation; or the aforementioned method including the step of co-culturing the heterogeneous stem cell population according to any one of claims 1-3 and isolated PBMCs in a culture medium containing PolylC or IFN-γ+TNF-α (I+T), thereby inhibiting T cell activation.

6. Use of the heterogeneous stem cell population according to any one of claims 1-3 in the preparation of a medicament for repairing tissue damage, preferably, the tissue damage is hepatocyte damage or kidney damage.

7. Use of the heterogeneous stem cell population according to any one of claims 1-3 in the preparation of a medicament for treating cGVHD.

8. A method for preparing the heterogeneous stem cell population according to any one of claims 1-3, including the following steps:
1) obtaining a stem cell population;
2) culturing the stem cell population obtained in step 1) in a culture medium containing 50%-99% DMEM/F12, 0.1-30 ng/ml epidermal growth factor, 0.1-2% B27 and 0.1-10% FBS.

9. Use of the heterogeneous stem cell population according to any one of claims 1-3 in the preparation of a medicament for intervening the blood-brain barrier.

10. Use of the heterogeneous stem cell population according to any one of claims 1-3 in the preparation of a medicament for regulating the metabolism of adipose cell tissue.

11. A method for inducing the heterogeneous stem cell population according to any one of claims 1-3 into an anti-inflammatory stem cell population *in vitro,* including contacting the heterogeneous stem cell population according to any one of claims 1-3 with the small molecule CZ, whose potential pathway is the mTOR-AKT-FOXO3-IDO signaling pathway axis, and eventually promoting the immunosuppression function of MSCs by promoting the transcription of IDO.

12. An anti-inflammatory stem cell population obtained by the method according to claim 11.

13. Use of the anti-inflammatory stem cell population obtained by the method according to claim 11 in the preparation of a medicament for treating autoimmune diseases.
